# EUROPEAN PATENT SPECIFICATION

(11) **EP 4 441 279 B1**
(45) Date of publication and mention of the grant of the patent: **29.10.2025**
(21) Application number: 22822506.6
(22) Date of filing: 28.11.2022
(51) Int. Cl.: D01F 9/00, D01F 9/22, D01F 9/32, F27B 9/28, F27B 9/06, F27B 9/30, G01N 25/48, F27D 11/12, F27D 19/00, F27D 21/02, D02J 13/00, G01N 33/36

(54) **CONTINUOUS OVEN**
DURCHLAUFOFEN
FOUR CONTINU

(30) Priority: 29.11.2021 EP 21211067
(43) Date of publication of application: 09.10.2024
(73) Proprietor: Teijin Carbon Europe GmbH, 42103 Wuppertal (DE)
(72) Inventor: WÖLKI, Michael, 65934 Frankfurt am Main (DE)
(74) Representative: CPW GmbH
(86) International application number: PCT/EP2022/083457
(87) International publication number: WO 2023/094650

(56) References cited:
- WO-A2-2011/094615
- JP-A- 2009 174 077
- US-A1- 2021 198 816

## Description

The application pertains to an oven and a method for oxidizing polyacrylonitrile precursor fibers in processes for manufacturing carbon fibers.

Carbon fibers have become an important material during the past 150 years. The first industrial use of carbon fibers may have been as incandescent wire in carbon filament light bulbs in the second half of the 19^{th} century. However in the second half of the 20^{th} century, not the electrical but the mechanical properties of high-performance carbon fibers are in the focus of interest as light-weight material of high tenacity. Typically, carbon fibers are used in compound materials embedded into a thermoplastic or thermosetting resin matrix, often known as carbon fiber reinforced plastic e.g. for the construction of airplane fuselages or wings. As an example, both the modern airliners Boeing 787 and Airbus A350 are to a large extent made from carbon fiber reinforced plastics. Also goods such as sports articles like skis, skiing poles, rackets for different kinds of sports like hockey or tennis and also whole boats are made from carbon fiber reinforced plastics. Carbon fibers furthermore are used as reinforcement for other materials than resin such as concrete.

In general, carbon fibers are produced from organic carbon-containing fibers in thermal processes. While for carbon filament light bulbs plant-based cellulose fibers from e.g. bamboo or cotton have been carbonised, a common modern precursor for carbon fibers is polyacrylonitrile, a typically petrol-based polymer. Other possible precursors are cellulose fibers such as rayon-based fibers obtained from e.g. the viscose or Lyocell processes or pitch.

From polyacrylonitrile (PAN), fibers are typically spun by precipitation of the polymer from a solution and dried. The dried fibers are the starting point for a very complex production process which consumes both a large amount of time and energy.

This process offers the potential for reduction in energy consumption and, therefore, in the improvement of the carbon dioxide footprint of carbon fibers.

The process of carbon fiber production from PAN precursor fibers in general consists of two steps, oxidation and carbonization. In the oxidation step, PAN undergoes a cyclization reaction forming chains of annealed nitrogen-containing six-membered rings and, afterwards, in an oxidative dehydrogenation reaction, annealed pyridine rings resulting in oxidized PAN fibers which are by themselves used as flame resistant unmeltable materials and which are further processed to carbon fibers. In the oxidation step from meltable PAN, unmeltable oxidized PAN is obtained. Furthermore, in the oxidation process, a lot of chemical energy is released from the precursor fibers and thus, the subsequent carbonization process can be carried out without significant heat emissions and is thus easy to control. While processing of carbon fibers from oxidized PAN fibers can be carried out in a comparably fast process in a carbonization furnace at temperatures above 300°C and up to 3000 °C under inert gas, the oxidation process is the time-determining step as it is highly exothermic and thus has to be carried out under well controlled conditions in order to make sure that the reaction does not run out of control which may lead to burning and thus destruction of the fiber, explosions in the oxidation oven and uncontrolled emission of highly toxic gasses such as carbon monoxide and hydrogen cyanide.

Typically, the oxidation process is carried out in an oven with several stages with every stage having a constant temperature and temperatures increase from stage to stage while first cyclization and afterwards oxidative dehydrogenation takes place. In the stages of the oven, temperatures have to be kept at a very constant level which makes sure that the cyclization and oxidative dehydrogenation reactions take place but that the reaction rate is kept at a level where only a limited amount of heat is emitted which cannot cause the reaction to run out of control. As for these reasons, the reaction rate has to be kept at a very low level, the oxidation step is the time-determining step in carbon fiber production which takes between 45 and 60 minutes to get from a meltable PAN fiber to an oxidized unmeltable PAN fiber.

Cellulose fibers as precursors are not meltable as PAN fibers are, however these fibers are also inflammable and thus, their requires thorough control of the reaction temperature. The same is the case for pitch as precursor for carbon fibers although oxidation of rayon-based and pitch-based precursor fibers is less exothermic. For all these precursors, the reaction is exothermic either in part or in total. In exothermic parts of the process, the temperature of the precursor fiber under reaction is higher than the temperature of the oven in which the reaction occurs.

Throughout this application, the term "precursor fiber" and "precursor thread" will be used synonymously. In general, both terms mean any kind of thread or fiber that can be used to produce carbon fibers thereof by thermal processing. This explicitly includes PAN-based precursor fibers, pitch-based precursor fibers and rayon-based precursor fibers. In the sense of the present application, rayon-based precursor fibers may also comprise lignin.

While passing through the oven, the at least one precursor fiber may undergo a chemical reaction which means that its chemical entity changes between entering and leaving the oven. A PAN precursor fiber or thread or any other precursor fiber or thread entering the oven according to the application may leave the oven as an oxidized. During passage of the oven, the fiber or thread may change its chemical entity from meltable PAN to oxidized unmeltable PAN, from inflammable rayon-based precursor fiber to rayon-based oxidized fiber or from pitch to oxidized pitch fiber through intermediate forms known to the person skilled in the art. Just for the ease of description, throughout this application, a PAN precursor fiber or thread or any other precursor fiber or thread passing through an oven according to the application is called a "precursor thread" or a " precursor fiber" independent of the degree of change of chemical entity towards an precursor fiber or thread. It is however understood that after passage of the oven, the thread or fiber is an oxidized precursor thread or fiber.

Further relevant information can be found in document WO 2011/094615, US 2021/198816 and JP 2009 174077.

It is the objective of the present application to ease the mentioned disadvantages by reducing the time and energy consumption of the production of carbon fibers. It has now surprisingly been found that these problems are solved by a continuous oven comprising a processing direction and at least one oven module which oven module has two openings positioned in such a way that at least one precursor fiber may pass through the oven module in the processing direction, the oven module further comprising at least one blower suitable for producing a gas flow perpendicular to the processing direction, at least one tunable radiative heater, preferably a microwave heater, radiating perpendicular to the processing direction for heating the at least one precursor fiber to a first temperature, at least one second heater, preferably an electrical resistance heater, for heating the gas within the oven to a second temperature, at least one optical detection means, preferably an infrared camera or an infrared spectrometer as well as at least one control unit, preferably a computer, characterized in that the blower, the radiative heater, the second heater, the optical detection means and the control unit serve as regulatory means for the heat emitted by the at least one precursor fiber in order to keep the temperature difference between the first temperature and the second temperature less than 10 °C.

An oven according to the application is any device comprising at least one opening wherein goods can be heated to a temperature which is typically above 100°C and for which there is essentially no upper limit. The source of heat may be radiation such as microwaves or infrared radiation, an electrical resistance heater or any kind of burning materials such as gas, oil, coal or wood. Similar devices are known to the skilled person under the terms "kiln" or "furnace". Throughout the present application, the term "oven" will constantly be used, however it is declared that wherever the term "oven" occurs, also the terms "kiln" or "furnace" may be used. Transfer of heat from the heat source to the oven may be by any technique known in the art such as placement of a device such as an electrical resistance heater in the cavity of the oven, heating the walls of the oven from the inside and/or the outside by an electrical resistance heater or by burning materials such as gas, oil, coal or wood. Furthermore, the oven according to the present invention may also be heated using an energy-carrier liquid such as a thermal oil, e.g. using lost heat from other processes such as e.g. carbonization furnaces wherein oxidized precursor fibers are processed into carbon fibers. Ovens using this heating technique are known to the skilled person as muffle ovens. Heat can also be transferred from the heat source to the oven using a transport medium such as air or any other gas or gas mixture or liquid such as thermal oil. The air, gas or gas mixtures may be moved by natural convection only or may be moved by forced convection by any suitable means known by those skilled in the art such as at least one ventilator, at least one pump and/or at least one turbine.

An oven module according to the present application is a part of an oven which may both serve as an oven by itself but which may also be combined with an unlimited number of other oven modules to form a composed oven. Throughout the present application, it is understood that the term "oven" can always mean an oven comprising only one oven module or an oven comprising two, three, four, five or any other number of oven modules. Throughout this application, the term "oven" can thus always mean an oven comprising one single module or an oven comprising more than one oven module. In an embodiment, the oven may consist of one oven module which then means that the oven module is the oven. Only when necessary, the term "composite oven" will be used for an oven of more than one module. Thus an oven according to the application always comprises one oven module, however the amount of oven modules comprised in an oven according to the application is not limited.

The oven modules comprised in one oven according to the application may be of the same shape or of different shapes.

In order to be suitable for connection with other oven modules, an oven module according to the present application may comprise at least one means for connection to another oven module such as e.g. at least one opening with the same size as the opening of another oven module and which may be combined in such a way that said openings connect the oven e.g. as part of a connecting tunnel. In an embodiment, the oven module may comprise more than one connection means in such a way that one oven module may be connected with either two or even more oven modules and thus form an oven comprising either a chain or even a network of oven modules.

In an embodiment, the means for connection may be openings in the oven module which are located in such a way that the at least one precursor fiber - may pass through the oven in the processing direction. The openings may also be interpreted as the inlet and the outlet of the oven module, respectively. In case the oven consists of only one oven module, the inlet and the outlet of the oven module are the inlet and the outlet of the entire oven, respectively. In case the oven comprises more than one oven module, the inlet of the oven is the inlet of one oven module and the outlet of the oven is the outlet of another oven module while the other inlets and outlets of the oven modules are connected to each other.

In an embodiment, several oven modules may be connected to one oven stage with several oven stages being combined to one composite oven.

When connected to each other, the connection means of the oven modules may be sealed in such a way that heat is blocked from leaving the oven.

In an embodiment, the oven modules may comprise connection means for electrical connections which may be plugs and/or sockets for one or more wires and/or cables which form a power line connection between the oven modules and/or a data connection between the oven modules.

In an embodiment, the oven modules may have the shape of tube or tunnel segments or domes comprising at least one opening for connection to other oven modules.

As any oven module according to the application can be an oven by itself, an oven module comprises all features of the oven according to the application such as at least one radiative heater, at least one second heater, at least one optical detection means and at least one control unit and within any oven module, said features serve as regulatory means for the heat emitted by the at least one precursor fiber in order to keep the difference between the first and the second temperature constant. The at least one radiative heater, the at least one second heater, the at least one optical detection means and the at least one control unit of any oven module can work separately from the heaters, optical detection means and control units of other oven modules of the same oven or the at least one heater, the at least one optical detection means and the at least one control unit of an oven module may be connected to the heaters, optical detection means and/or control units of at least one, several or all other oven modules. In an embodiment, a composite oven according to the present application further comprises a central control unit for the whole oven which may be independent from the control units of the oven modules or which may be connected to one, several or all control units of one, several or all oven modules.

In an embodiment, the modules forming the oven are similar or identical which then allows for easily exchanging a module in case of a defect.

The oven according to the application is a continuous oven which means that the at least one precursor fiber within the oven is moving either constantly or with interruptions while being heated. This implies that the oven has an elongated shape which may be the shape of a tunnel, a tube, a slot or a duct through which the at least one precursor fiber is passing. The direction in which the at least one precursor fiber is passing is called the processing direction. The processing direction can be any direction. It can be horizontal e.g. in a tunnel-shaped oven or vertical e.g. in a duct-shaped oven. The processing direction can also be inclined or even follow a curved path.

Along the processing direction, the conditions in the oven such as the second temperature, gas circulation or partial pressure of gasses such as nitrogen, oxygen or carbon dioxide may be constant or variable. In an embodiment, said conditions are constant within one oven module and may change between different modules. Constant according to the application means that a condition such as temperature, gas flow or gas partial pressure does not change by more than 5%.

The chemical reaction of the at least one precursor fiber while passing the oven is exothermic, the temperature of the at least one precursor fiber will thus be higher than the second temperature. In this case, the chemical reaction yields energy while the heat of the oven just supplies the activation energy for the reaction.

The second heater according to the present application is able to heat the gas flow within the oven to a second temperature. In an embodiment, the second heater is an electrical resistance heater comprising a heat exchanger for transferring heat to the gas flow within the oven module. The oven module according to the application and the oven comprising said oven module may, according to this embodiment, be considered to be a hot-air oven or a hot-air oven module, respectively.

In an embodiment, the second heater is located out of the oven module and heats the walls of the oven module in the fashion of a muffle oven. In such case, the second heater may be an electrical resistance heater or a burning material such as wood, coal, oil or gas. The oven module according to this embodiment may work in the manner of a muffle oven. In such a case the gas flow within the oven is not heated directly but by the walls of the oven. The main purpose of the gas flow is then to equally distribute the heat within the oven.

Throughout the present application, the term "second temperature" will be used synonymously to the term "oven temperature". The second temperature is the temperature of the air flow within the oven which, as the reaction is exothermic is **lower or equal** to the first temperature.

The second temperature may vary through the oven. In an embodiment, every oven module has its own constant second temperature. In an embodiment, the second temperature varies throughout an oven module.

The number of precursor fibers passing the oven is not particularly limited and depends only on the size of both the oven and the size of the precursor fibers. Precursor fibers or bundles of precursor fibers may pass the oven in parallel. In an embodiment, several hundreds or even several thousands of precursor fibers may pass the oven in parallel.

A blower according to the application is any device which can cause an air or gas flow. In an embodiment, the blower is a fan such as an axial fan, a centrifugal fan or cross-flow fan. The blower may further be a fan utilizing the Coanda effect. The gas flow generated by a blower is directed. In an embodiment, the blower may contain a means for heating or cooling the air or gas flow generated by the blower. The blower according to the application generates a gas flow which is essentially perpendicular to the processing direction.

Throughout this application, the term "essentially perpendicular to the processing direction" means that an angle between 80° and 100° with the processing direction is formed.

A radiative heater according to the application is any device that emits electromagnetic radiation suitable for heating an object. In an embodiment, the radiative heater may be a microwave heater. A microwave heater is a device such as a cavity magnetron which emits electromagnetic radiation at a wavelength between 30 cm and 1 mm which is able to excite molecules to rotationally excited states and thus is able to heat objects.

In an embodiment, the heater is an infrared radiation source. Infrared radiation is electromagnetic radiation at a wavelength between 1 mm and 780 nm which is able to excite molecules to vibrationally excited states and to thus heat objects. Possible infrared radiation sources are e.g. a Nernst lamp, a globar, a heat lamp such as a quartz heat lamp or a carbon heater.

In an embodiment, the heater is a laser emitting either infrared or visible light. The heater is suited and installed in such a way that it emits radiation essentially perpendicular to the processing direction.

The heater is able to heat the at least one precursor fiber to a first temperature. Throughout this application, the term "first temperature" will be used synonymously with the term "temperature of the at least one precursor fiber".

The first temperature may vary upon passage of the at least one precursor fiber through the oven. If the at least one precursor fiber undergoes a chemical reaction during passage through the oven, the first temperature at a position in the oven may depend on the chemical entity of the at least one precursor fiber at said position.

Using the radiative heater, the second heater, the blower, the optical detection means and the control unit, it is possible to keep the difference between the first and the second temperature essentially constant this means the temperature difference between the first and the second temperature differs less than 20 °C. In an embodiment, the two temperatures differ by no more than 10°C, 7°C, 5°C, 3 °C or 2°C.

In an embodiment, the second temperature is always 20°C or 10°C lower than the decomposition temperature of the oxidized precursor thread.

In order to keep the temperature difference essentially constant, only the first temperature, only the second temperature or both temperatures may be varied. In order to keep the temperature difference essentially constant, the first and the second temperature may either both be kept constant or the first and the second temperature may be varied in temperature sequences with the same shape which are only shifted against each other by the essentially constant difference between the first and the second temperature. Using temperature sequences like this, the first and the second temperature may e.g. be constantly increasing during passage through the oven.

The optical detection means according to the application is a device that is able to receive and to measure electromagnetic radiation and to analyze said electromagnetic radiation with respect to both its intensity and its spectral distribution.

The optical detection means may be sensitive in the infrared region of the electromagnetic spectrum (1 mm - 780 nm wavelength), in the visible region of the electromagnetic spectrum (780 nm - 300 nm) or in the ultraviolet region of the electromagnetic spectrum (300 nm - 100 nm) or in combinations thereof. The optical detection means may further comprise a radiation source emitting a kind of radiation the optical detection means is able to detect such as an infrared lamp or a Nernst lamp, a globar, a heat lamp such as a quartz heat lamp or a carbon heater, a light bulb, light emitting diode or fluorescent lamp emitting visible or ultraviolet light or any combination thereof. In contrast to the radiative heater which has the main purpose of heating the at least one precursor fiber, the radiation source comprised in the optical detection means should be able to emit radiation with a broad distribution of wavelength in order to serve as a light source for a spectrometer.

The optical detection means may be a pyrometer, a camera or a spectrometer. Using a pyrometer, the temperature of a distant object can be measured by detection of its infrared radiation.

Using a camera, an image of an object can be obtained wherein the properties such as wavelength and intensity of radiation emission of the at least one precursor fiber can be measured not only for a single spot but in a two-dimensional manner. This allows e.g. to get a two-dimensional picture of the color and/or the temperature of the at least one precursor fiber.

A spectrometer is a device which is able to decompose a ray of electromagnetic radiation with respect to the wavelengths comprised therein and to thus measure the spectral distribution of electromagnetic radiation which does not only allow to conclude on the temperature of the at least one precursor fiber but also to conclude on its chemical composition. Using a spectrometer, which may be an infrared and/or a visible and/or a UV spectrometer, the chemical reaction the at least one precursor fiber in the oven is undergoing can thus be monitored.

A control unit according to the application may be any electronic device that is able to receive and evaluate measured data and to regulate other devices. In an embodiment, the control unit is a computer.

The at least one control unit is connected to the at least one radiative heater, the at least one blower and the at least one optical detection means and is able to evaluate data received therefrom and to regulate both the radiative heater and the blower in dependency of the data obtained which may be data concerning the first and/or the second temperature, data concerning the chemical composition of the at least one precursor fiber and thus data on the state of the chemical reaction the at least one precursor fiber is undergoing or any combination thereof.

The oven may comprise at least one guiding means for threads. A guiding means for threads according to the application may be at least one pulley, at least one loop, at least one comb or at least one godet or any combination thereof. The oven may also comprise a multiple of guiding means for threads in such a way that several threads, e.g. several hundred or several thousand threads, may pass through the oven in parallel.

In an embodiment, the means for guiding threads is located at the boundaries between modules of the oven.

The guiding means for threads may be equipped with force sensors which allow for measuring the drawing forces of the threads being passed over the guiding means for threads. Measuring of the drawing forces may provide another source of information on the chemical reaction the thread is undergoing upon passing through the oven. In a chemical reaction the thread may shrink or elongate and the amount of shrink or elongation may allow for conclusion on the rate of the chemical reaction. The data about shrink or elongation may supplement the data obtained by the optical detection device on the temperature and/or chemical composition of the thread and may thus also be used for regulating the blower and/or the heater.

The oven according to the present application may comprise paddles which are suitable for guiding a gas flow within the oven. Said paddles may be mounted in such a way that they are movable and that their movements can be controlled by the control device.

In an embodiment, at least one blower is a turboblower.

In an embodiment, at least one blower comprises a nozzle which is directed in such a way that a gas flow can be formed which is essentially perpendicular to the processing direction. Said nozzle may also be adjustable in both width and direction and can thus be used to modify the gas flow within the oven. Said adjustable nozzle may be controlled by the control unit.

In an embodiment, the oven according to the application may comprise at least one nozzle which is connected to an inert gas reservoir such as a bomb or a tank comprising nitrogen, carbon dioxide, argon or any other inert gas. The nozzle may then be used to selectively lower the partial pressure of oxygen at the at least one precursor fiber and at the same time to transport away heat from the at least one precursor fiber in order to avoid the at least one precursor fiber to undergo an uncontrolled oxidation reaction such as burning. Thus the effect of the nozzle emitting inert gas may be compared to the effect of a gas-based fire extinguisher. The application further pertains to a process for oxidizing PAN precursor threads or other precursor threads comprising a self-learning artificial neural network.

An artificial neural network is composed of artificial neurons. An artificial neuron is a software structure which is able to take input values, multiply them with weight factors and - from these weighted inputs - generates a network input value which is supplied to an activation function which generates the neuron output which may be a number or a logical value ("true" or "false").

Self-learning artificial neural networks can be trained using training sets of data which training sets comprise both input and desired ("correct") output values. In algorithms known to the person skilled in the art within the neurons of the neural network, the weight factors can be adapted such that the artificial neural network can, from the provided training set, learn to produce the desired output values and to afterwards evaluate similar input values.

Throughout this application, the term "self-learning artificial neural network" means all structures of artificial neurons which can be trained to produce a reasonable output from a given input. It is explicitly noted that also one isolated artificial neuron is considered to be a self-learning artificial neural network as long as it has the ability of adapting its input weights.

The number of neurons comprised in the self-learning artificial neural network according to the application is essentially not limited. A typical self-learning artificial neural network comprises artificial neurons in one or more so-called layers with different functionalities. One layer e.g. typically serves as input layer which receives input data e.g. from sensors or computers which generate input data for the artificial neural network. One layer of the artificial neural network produces the output. Any artificial neuron of the output layer may produce an output in form of either a logical value or a number. Note that the output layer and the input layer of an artificial neural network may be the same layer. In such case, a one-layer artificial neural network is present.

In between the input layer and the output layer, the artificial neural network may comprise further layers of neurons which are connected e.g. to all or a part of the input neurons, to all or a part of the output neurons and/or to all or a part of further neurons which are not part of the input or output layers. Layers of artificial neurons which neither receive input from outside the artificial neural network nor produce an output outward the artificial neural network are called "hidden layers" and the neurons comprised therein are called "hidden neurons".

In case connections between the neurons of an artificial neural network are only between neurons of adjacent layers, such as e.g. between neurons of the input layer and a first hidden layer, between neurons of the first hidden layer and a second hidden layer and between neurons of the second hidden layer and the output layer, the artificial neural network is called a feedforward artificial neural network. In a feedforward artificial neural network, information can only be transferred from the input layer towards the output layer. An important type of feedforward artificial neural network is known to the skilled person as perceptron. Artificial neural networks can also be built in such a way that an artificial neuron can receive input either from itself, from neurons of the same layer or from neurons of a layer which is closer to the output layer than the neuron itself. Artificial neural networks of said shape, wherein information is not transferred straightforwardly towards the output layer but can be transferred in so-called feedback loops are known to the skilled person as recurrent artificial neural networks. For the presence of feedback loops, recurrent artificial neural networks can develop a memory.

In the process according to the present application, all kinds of artificial neural networks may be used. In an embodiment, the artificial neural network used therein is a perceptron. In an embodiment, the artificial neural network used therein is a recurrent artificial neural network. In an embodiment, the neural network used is an adaptive linear neural network (ADALINE).

The key functionality of the artificial neural network used in the process according to the present application is to control the oxidation process of the at least one precursor fiber.

Due to its highly exothermic character, the oxidation process of precursor threads requires strict control of the temperature of the threads undergoing the oxidation reaction in order to prevent the threads from burning or even exploding due to heat accumulation in fiber bundles. In current processing techniques, control of the temperature of the threads is carried out by a control of the temperature of the oven the precursor threads are passing through. The temperature of the oven is thereby kept at the lower edge of the temperature window in which the desired reaction takes place at all in order to avoid the precursor threads to catch fire due to the highly exothermic oxidation process. Control of said process e.g. by a person permanently observing the precursor threads would not be possible at an efficient level as in an oxidation oven hundreds or even thousands of fibers are processed at the same time making it impossible for one person or a reasonable amount of persons to observe and to manually control the oxidation process. There is thus no current alternative to keep the temperature of the threads at the lower edge of the temperature window in which the desired reaction takes place. Typically, the oven used for such processes comprises several stages which are held at different constant temperatures. A precursor thread entering a stage start to react as the heat of the oven stage serves as activation energy to the oxidation reaction causing the thread to warm up above the temperature of the oven stage. The temperature of the oven stage is chosen in such a way that the oxidation reaction is initiated in such a way that it cannot run out of control and that during passage of the oven module, the reaction rate decreases and the temperature of the fiber decreases resulting in a low reaction rate and thus in a long reaction time. Using the oven according to the present application, control of the reaction becomes possible using an artificial neural network or several artificial neural networks. In the process according to the present application, artificial neural networks are provided with data from the at least one optical detection means and/or the sensors on the guiding means for the threads. Said data may be data on the temperature of the threads, the temperature of the oven and/or the current chemical composition of the oxidizing precursor thread or data on the shrink or expansion of the precursor thread.

In an embodiment, said data are recorded for every precursor thread individually and can be fed into the artificial neural network together with global data such as data on the temperature within the oven or oven module or the rate and direction of gas flow within the oven or oven module. The artificial neural network used in the process according to the application may thus comprise at least as many input neurons as precursor threads are passed through the oven.

The artificial neural network can then be trained to calculate from the input data how the temperature of the threads and/or the temperature of the oven may be changed in order to keep the difference between the first and the second temperature essentially constant. The artificial neural network should thus comprise at least as many output neurons as there are precursor threads passing through the oven or the oven module.

Depending on the output data of the neural network, the temperature of any individual precursor thread may be increased or reduced using the blower and/or the heater in order to keep the difference between the first and the second temperature essentially constant. Using the artificial neural network, control of the oxidation process can be strongly intensified and can thus be carried out at much higher reaction rate compared to the state of the art.

The present application further pertains to a process for oxidizing polyacrylonitrile precursor threads comprising the steps of providing polyacrylonitrile precursor threads, providing an oven according to present application, drawing and spreading the PAN precursor threads, passing the PAN precursor threads through the oven, controlling the amount of heat being emitted by the oxidizing PAN precursor threads, regulating the amount of heat emitted by the oxidizing PAN precursor threads using the heater and the blower, winding up the oxidized polyacrylonitrile precursor threads.

The present application further pertains to a process for oxidizing precursor threads comprising the steps of providing precursor threads, providing an oven according to present application, spreading and optionally drawing the precursor threads, passing the precursor threads through the oven, controlling the amount of heat being emitted by the oxidizing and/or carbonizing precursor threads, regulating the amount of heat emitted by the oxidizing precursor threads using the heater and the blower, winding up the oxidized and/or carbonized precursor threads.

In an embodiment, for regulating the amount of heat emitted by the oxidizing precursor threads, the blower is able to blow inert gas such as nitrogen or carbon dioxide, into the oven.

In an embodiment, the oxidizing precursor threads are selectively heated or cooled depending on their temperature.

In an embodiment, regulation of the process is based on a self-learning artificial neural network, preferably a recurrent self-learning artificial neural network.

### Description of the Figures

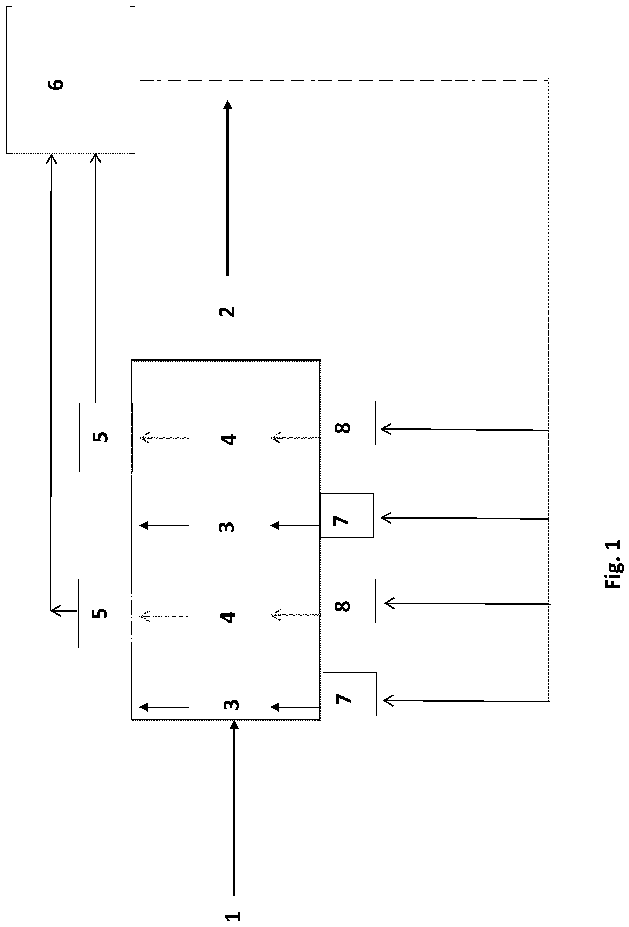

**Fig. 1** shows a schematic view of an oven module according to the application with an inlet for precursor threads 1 and an outlet for oxidized precursor threads 2. Within the oven, a gas flow 3 is initiated by blowers 7 and infrared radiation 4 emitted by radiative heaters 8 is irradiated towards the threads within the oven module. Infrared radiation is detected by spectrometers 5 which are connected to a control unit 6 which controls the blowers and the radiative heaters. The blower may also comprise the second heater (not shown).

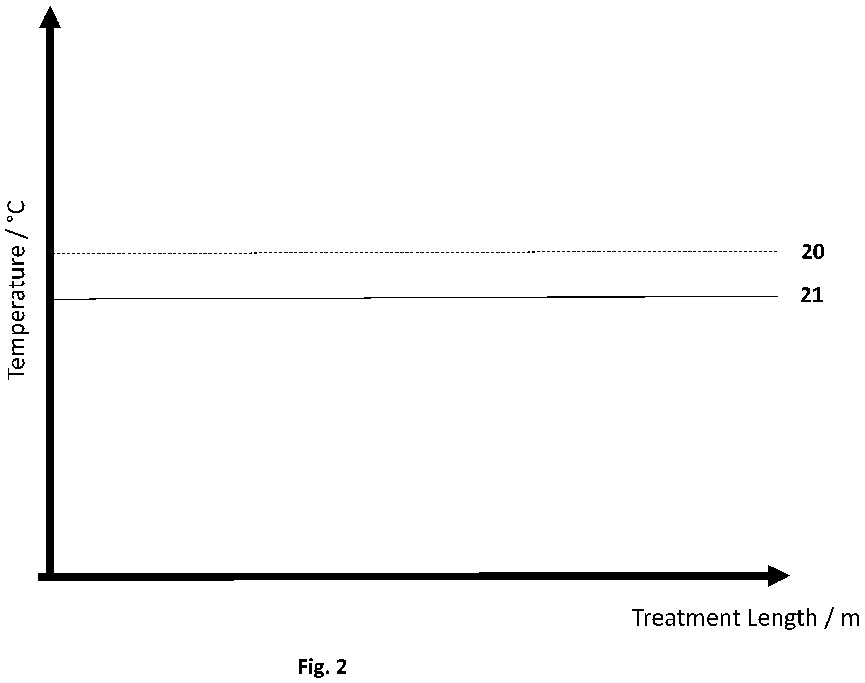

**Fig. 2** shows the temperature of the gas flow 21 and the precursor or oxidized precursor thread 20 during passage through an oven module according to the application.

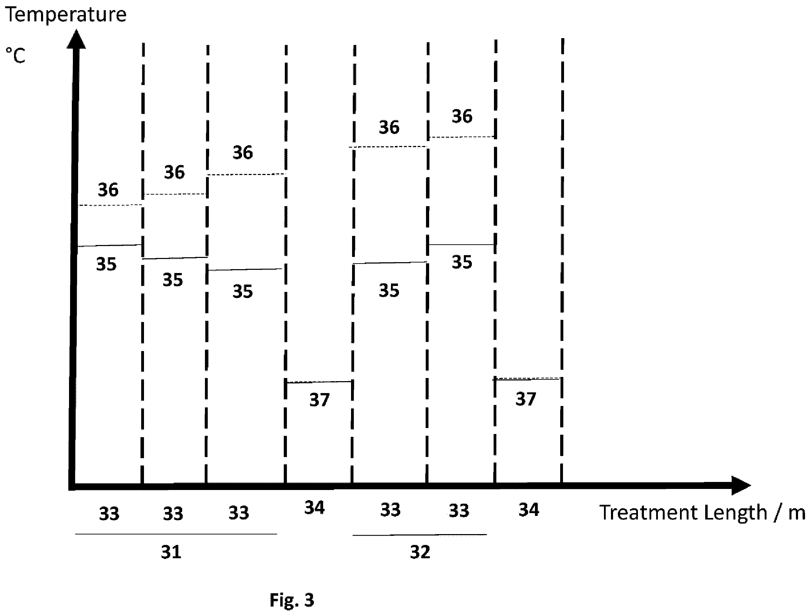

**Fig. 3** shows a schematic image of an embodiment of the oven according to the application comprising a first stage 31 and a second stage 32 with the first stage comprising three oven modules 33 according to the application and the second stage comprising two oven modules 33 according to the application. In between the stages, yarn transport units 34 are installed. The gas flow within every oven module is kept at a constant temperature level 35 while using the radiative heater, the blower and the control unit of the oven modules, the temperature level of the PAN precursor thread (not shown) within the oven module is kept at a constant temperature level 36 which is above the constant temperature level 35. In between the stages, while passing the yarn transport unit, the temperature of the precursor yarn is at the temperature outside the oven 37.

## Claims

1. Continuous oven comprising a processing direction and at least one oven module which oven module has two openings positioned in such a way that at least one precursor fiber may pass through the oven module in the processing direction, the oven module further comprising at least one blower suitable for producing a gas flow perpendicular to the processing direction, at least one tunable radiative heater, preferably a microwave heater, radiating perpendicular to the processing direction for heating the at least one precursor fiber to a first temperature, at least one second heater, preferably an electrical resistance heater, for heating the gas within the oven to a second temperature, at least one optical detection means, preferably an infrared camera or an infrared spectrometer as well as at least one control unit, preferably a computer, **characterized in that** the blower, the radiative heater, the second heater, the optical detection means and the control unit serve as regulatory means for the heat emitted by the at least one precursor fiber in order to keep the temperature difference between the first temperature and the second temperature less than 10 °C with the first temperature being higher than the second temperature.

2. Oven according to claim 1 wherein the oven comprises several oven modules.

3. Oven according to any one or more of the preceding claims wherein the oven at the boundary between at least two modules comprises guiding means for threads such as pulleys, loops, combs or godets.

4. Oven according to claim 3 wherein the drawing force of the guiding means for threads can be measured and may additionally be used as regulatory means for the heat emitted by the at least one precursor fiber.

5. Oven according to any one or more of the previous claims wherein the at least one precursor fiber is at least one thread of polyacrylonitrile precursor thread.

6. Oven according to any one or more of the preceding claims wherein the radiative heater is an infrared laser.

7. Oven according to any one or more of the preceding claims wherein the oven comprises paddles suitable for guiding the gas flow within the oven.

8. Oven according to any one or more of the previous claims wherein at least one blower is a turbo blower.

9. Oven according to any one or more of the previous claims wherein the at least one blower comprises a nozzle which is directed into the oven.

10. Process for oxidizing precursor threads comprising at least one self-learning artificial neural network.

11. Process according to claim 10 wherein at least one self-learning artificial neural network is a recurrent neural network.

12. Process for oxidizing polyacrylonitrile precursor threads comprising the steps of
a. providing polyacrylonitrile precursor threads,
b. providing an oven according to any one or more of the preceding claims,
c. drawing and spreading the polyacrylonitrile precursor threads,
d. passing the polyacrylonitrile precursor threads through the oven,
e. controlling the amount of heat being emitted by the oxidizing polyacrylonitrile precursor threads,
f. regulating the amount of heat emitted by the oxidizing polyacrylonitrile precursor threads using the heater and the blower,
g. winding up the oxidized polyacrylonitrile precursor threads.

13. Process according to claim 12 wherein the blower for regulating the amount of heat emitted by the oxidizing polyacrylonitrile precursor threads is able to blow inert gas, preferably nitrogen or carbon dioxide, into the oven.

14. Process according to any one or more of claims 12 and 13 wherein the oxidizing polyacrylonitrile precursor threads are selectively heated or cooled depending on their temperature.

15. Process according to any one or more of claims 12 to 14 wherein the regulating of the process is based on at least one algorithm or at least one self-learning artificial neural network, preferably a recurrent self-learning artificial neural network.

## Patentansprüche

1. Durchlaufofen, umfassend eine Verarbeitungsrichtung und mindestens ein Ofenmodul, wobei das Ofenmodul zwei Öffnungen aufweist, die so positioniert sind, dass mindestens eine Ausgangsfaser in der Verarbeitungsrichtung das Ofenmodul passieren kann, wobei das Ofenmodul weiter mindestens ein Gebläse umfasst, das geeignet ist, um eine Gasströmung rechtwinkelig zu der Verarbeitungsrichtung zu erzeugen, mindestens eine einstellbare Strahlungsheizvorrichtung, bevorzugt eine Mikrowellenheizvorrichtung, die rechtwinkelig zu der Verarbeitungsrichtung strahlt, um die Ausgangsfaser auf eine erste Temperatur zu erwärmen, mindestens eine zweite Heizvorrichtung, bevorzugt eine elektronische Widerstandsheizung zum Erwärmen des Gases innerhalb des Ofens auf eine zweite Temperatur, mindestens ein optisches Detektionsmittel, bevorzugt eine Infrarotkamera oder ein Infrarotspektrometer sowie mindestens eine Steuereinheit, bevorzugt einen Computer, **dadurch gekennzeichnet, dass** das Gebläse, die Strahlungsheizvorrichtung, die zweite Heizvorrichtung, das optische Detektionsmittel und die Steuereinheit als Regulierungsmittel für die von der mindestens einen Ausgangsfaser abgegebenen Wärme dienen, um den Temperaturunterschied zwischen der ersten Temperatur und der zweiten Temperatur bei weniger als 10 °C zu halten, wobei die erste Temperatur höher ist als die zweite Temperatur.

2. Ofen nach Anspruch 1, wobei der Ofen mehrere Ofenmodule umfasst.

3. Ofen nach einem oder mehreren der vorstehenden Ansprüche, wobei der Ofen an der Grenze zwischen mindestens zwei Modulen Führungsmittel für Fäden umfasst, wie Rollen, Schlaufen, Kämme oder Galetten.

4. Ofen nach Anspruch 3, wobei die Zugkraft der Führungsmittel für Fäden gemessen und zusätzlich als Regulierungsmittel für die von der mindestens einen Ausgangsfaser abgegebene Wärme verwendet werden kann.

5. Ofen nach einem oder mehreren der vorstehenden Ansprüche, wobei die mindestens eine Ausgangsfaser mindestens ein Faden aus Polyacrylnitril-Ausgangsfaden ist.

6. Ofen nach einem oder mehreren der vorstehenden Ansprüche, wobei die Strahlungsheizvorrichtung ein Infrarotlaser ist.

7. Ofen nach einem oder mehreren der vorstehenden Ansprüche, wobei der Ofen Schaufeln umfasst, die geeignet sind, um die Gasströmung innerhalb des Ofens zu leiten.

8. Ofen nach einem oder mehreren der vorstehenden Ansprüche, wobei mindestens ein Gebläse ein Turbogebläse ist.

9. Ofen nach einem oder mehreren der vorstehenden Ansprüche, wobei das mindestens eine Gebläse eine Düse umfasst, die in den Ofen gerichtet ist.

10. Prozess zum Oxidieren von Ausgangsfäden, der mindestens ein selbstlernendes künstliches neuronales Netzwerk umfasst.

11. Prozess nach Anspruch 10, wobei mindestens ein selbstlernendes künstliches neuronales Netzwerk ein rekurrentes neuronales Netzwerk ist.

12. Prozess zum Oxidieren von Polyacrylnitril-Ausgangsfäden, der folgende Schritte umfasst:
a. Bereitstellen von Polyacrylnitril-Ausgangsfäden,
b. Bereitstellen eines Ofens, nach einem oder mehreren der vorstehenden Ansprüche,
c. Ziehen und Ausbreiten der Polyacrylnitril-Ausgangsfäden,
d. Durchführen der Polyacrylnitril-Ausgangsfäden durch den Ofen,
e. Steuern der Menge von Wärme, die von den oxidierenden Polyacrylnitril-Ausgangsfäden abgegeben wird,
f. Regeln der Menge von Wärme, die von den oxidierenden Polyacrylnitril-Ausgangsfäden abgegeben wird, unter Verwendung der Heizvorrichtung und des Gebläses,
g. Aufwickeln der oxidierten Polyacrylnitril-Ausgangsfäden.

13. Prozess nach Anspruch 12, wobei das Gebläse zum Regulieren der Menge von Wärme, die von den oxidierenden Polyacrylnitril-Ausgangsfäden abgegeben wird, fähig ist, in den Ofen Inertgas, bevorzugt Stickstoff oder Kohlendioxid, hineinzublasen.

14. Prozess nach einem oder mehreren der Ansprüche 12 und 13, wobei die oxidierenden Polyacrylnitril-Ausgangsfäden abhängig von ihrer Temperatur wahlweise erwärmt oder abgekühlt werden.

15. Prozess nach einem oder mehreren der Ansprüche 12 bis 14, wobei das Regulieren des Prozesses auf mindestens einem Algorithmus oder mindestens einem selbstlernenden künstlichen neuronalen Netzwerk, bevorzugt einem rekurrenten selbstlernenden künstlichen neuronalen Netzwerk basiert.

## Revendications

1. Four continu comprenant une direction de traitement et au moins un module de four, lequel module de four présente deux ouvertures positionnées de telle sorte qu'au moins une fibre précurseur puisse passer à travers le module de four dans la direction de traitement, le module de four comprenant en outre au moins une soufflante adaptée à produire un écoulement gazeux perpendiculairement à la direction de traitement, au moins un élément chauffant radiatif réglable, de préférence un élément chauffant à micro-ondes, rayonnant perpendiculairement à la direction de traitement pour chauffer ladite au moins une fibre précurseur à une première température, au moins un second élément chauffant, de préférence un élément chauffant à résistance électrique, pour chauffer le gaz à l'intérieur du four à une seconde température, au moins un moyen de détection optique, de préférence une caméra infrarouge ou un spectromètre infrarouge, ainsi qu'au moins une unité de commande, de préférence un ordinateur, **caractérisé en ce que** la soufflante, l'élément chauffant radiatif, le second élément chauffant, le moyen de détection optique et l'unité de commande servent de moyens de régulation de la chaleur émise par ladite au moins une fibre précurseur afin de maintenir la différence de température entre la première température et la seconde température inférieure à 10 °C, la première température étant supérieure à la seconde température.

2. Four selon la revendication 1, dans lequel le four comprend plusieurs modules de four.

3. Four selon l'une quelconque des revendications précédentes, dans lequel le four à la limite entre au moins deux modules comprend des moyens de guidage de fils tels que des poulies, des boucles, des peignes ou des godets.

4. Four selon la revendication 3, dans lequel la force d'étirage des moyens de guidage de fils peut être mesurée et peut être utilisée en plus comme moyen de régulation de la chaleur émise par ladite au moins une fibre précurseur.

5. Four selon l'une quelconque ou plusieurs des revendications précédentes, dans lequel ladite au moins une fibre précurseur est au moins un fil de précurseur de polyacrylonitrile.

6. Four selon l'une quelconque des revendications précédentes, dans lequel l'élément chauffant radiatif est un laser infrarouge.

7. Four selon l'une quelconque des revendications précédentes, dans lequel le four comprend des palettes adaptées à guider l'écoulement gazeux à l'intérieur du four.

8. Four selon l'une quelconque ou plusieurs des revendications précédentes, dans lequel au moins une soufflante est une turbo soufflante.

9. Four selon l'une quelconque ou plusieurs des revendications précédentes, dans lequel ladite au moins une soufflante comprend une buse qui est dirigée dans le four.

10. Procédé destiné à oxyder des fils précurseurs comprenant au moins un réseau neuronal artificiel d'auto-apprentissage.

11. Procédé selon la revendication 10, dans lequel au moins un réseau neuronal artificiel d'auto-apprentissage est un réseau neuronal récurrent.

12. Procédé destiné à oxyder des fils précurseurs de polyacrylonitrile, comprenant les étapes destinées à
a. fournir des fils précurseurs de polyacrylonitrile,
b. fournir un four selon l'une quelconque ou plusieurs des revendications précédentes,
c. étirer et étaler les fils précurseurs de polyacrylonitrile,
d. faire passer les fils précurseurs de polyacrylonitrile à travers le four,
e. surveiller la quantité de chaleur émise par les fils précurseurs de polyacrylonitrile oxydant,
f. réguler la quantité de chaleur émise par les fils précurseurs de polyacrylonitrile oxydant en utilisant l'élément chauffant et la soufflante,
g. enrouler les fils précurseurs de polyacrylonitrile oxydés.

13. Procédé selon la revendication 12, dans lequel la soufflante destinée à réguler la quantité de chaleur émise par les fils précurseurs de polyacrylonitrile oxydant est capable de souffler un gaz intermédiaire, de préférence de l'azote ou du dioxyde de carbone, dans le four.

14. Procédé selon l'une quelconque ou plusieurs des revendications 12 et 13, dans lequel les fils précurseurs de polyacrylonitrile oxydant sont chauffés ou refroidis de manière sélective en fonction de leur température.

15. Procédé selon l'une quelconque ou plusieurs des revendications 12 à 14, dans lequel la régulation du procédé est basée sur au moins un algorithme ou au moins un réseau neuronal artificiel d'auto-apprentissage, de préférence un réseau neuronal artificiel d'auto-apprentissage récurrent.
